# EUROPEAN PATENT APPLICATION

(11) **EP 4 502 163 A1**
(43) Date of publication of application: **05.02.2025**
(21) Application number: 23775353.8
(22) Date of filing: 24.03.2023
(51) Int. Cl.: C12N 15/82

(54) **ADENINE-TO-GUANINE BASE EDITING METHOD OF PLANT CELL ORGANELLE DNA**

(30) Priority: 25.03.2022 KR 20220037211
(71) Applicant: Institute for Basic Science, Yuseong-gu Daejeon 34126 (KR)
(72) Inventor: KIM, Jin-Soo, Seoul 08831 (KR); MOK, Young Geun, Seoul 08833 (KR); BAE, Su-Ji, Daejeon 34046 (KR)
(74) Representative: Mewburn Ellis LLP
(86) International application number: PCT/KR2023/003940
(87) International publication number: WO 2023/182858

(57) **Abstract**

The present invention relates to a composition for base editing of plant cell organelle DNA and, in particular, to a composition and editing method for adenine-to-guanine editing of plant cell organelle DNA.

## Description

### [Technical Field]

The present invention relates to a composition for base editing of plant cell organelle DNA, and particularly to a composition and method for editing adenine to guanine in plant cell organelle DNA.

### [Background Art]

A fusion protein with a DNA binding protein and a deaminase linked enables single-nucleotide conversion in a targeted manner to perform targeted nucleotide substitution or base editing in the genome without generating DNA double-strand breaks (DSBs), to edit point mutations causing genetic disorders, or to introduce desired single-nucleotide variation in prokaryotes, humans, and other eukaryotes.

Program (set) genome editing tools such as ZFN (zinc finger nuclease), TALEN (transcription activator-like effector nuclease), CRISPR (clustered regularly interspaced short palindromic repeat) systems and CRISPR-associated protein 9 (Cas9) variants without nucleolytic efficiency, and base editing technology of nucleotide deaminase proteins have been developed for genetic research of plants and improvement of crop traits through changes in base sequences. However, these tools are not suitable for editing DNA sequences in plant organelles, including mitochondria and chloroplasts, mainly because of difficulties in delivering guide RNAs to organelles or co-expressing two compounds in organelles. Plant cell organelles encode many essential genes required for photosynthesis and respiration. Methods or tools for editing organelle genes are very necessary for studying the function of the genes or improving crop productivity and traits. For example, targeted mutations in the mitochondrial atp6 gene may lead to male sterility, a trait useful in reproduction, and specific point mutations in the 16S rRNA gene of the chloroplast genome may lead to antibiotic resistance.

Bacterial toxin DddAₜₒₓ is an enzymatic moiety in bacterial toxins derived from *Burkholderia cenocepacia* and is responsible for cytosine deamination within double-stranded DNA. As an example of a deaminase, DddAₜₒₓ is toxic in cells, and thus, to avoid toxicity in host cells, DddAₜₒₓ is split into two inactive halves, each of which is linked to a DNA binding protein designed to bind to DNA to make a functional DdCBE pair.

Against this technical background, it has been confirmed that adenine in plant cell organelle DNA may be edited to guanine, culminating in the present invention.

### [Disclosure]

It is an object of the present invention to provide a composition for editing the adenine base of plant cell organelle DNA, which includes a DNA binding protein, a cytosine deaminase in split form, and an adenine deaminase or a nucleic acid encoding the same.

It is another object of the present invention to provide a method of editing adenine to guanine in plant cell organelle DNA.

It is still another object of the present invention to provide an herbicide-resistant plant in which the adenine base of a chloroplast psbA gene is edited to guanine by the method described above.

It is yet another object of the present invention to provide a spectinomycin-resistant plant in which the adenine base of 16s rRNA is edited to guanine by the method described above.

It is still yet another object of the present invention to provide a plant having an albino phenotype in which the adenine base of a psaA gene is edited to guanine by the method described above.

In order to accomplish the above objects, the present invention provides a composition for editing the adenine base of plant cell organelle DNA, including a DNA binding protein or a nucleic acid encoding the same, a first split and a second split derived from a cytosine deaminase or a nucleic acid encoding the same, and an adenine deaminase or a nucleic acid encoding the same, in which each of the first split and the second split binds to the DNA binding protein.

The present invention also provides a method of editing adenine to guanine in plant cell organelle DNA, including treating a plant cell with the composition described above.

The present invention also provides an atrazine herbicide-resistant plant in which the adenine base of a chloroplast psbA gene including the GTTGAAAGC sequence is edited to guanine by the method described above.

The present invention also provides a spectinomycin-resistant plant in which the adenine base of 16s rRNA including the CGTCATCCTCA sequence is edited to guanine by the method described above.

The present invention also provides a plant having an albino phenotype in which the adenine base of a psaA gene including ATG is edited to guanine by the method described above.

### [Description of Drawings]

FIG. 1 schematically shows an editor in which DddAtox and adenine deaminase are attached to TALE;
FIG. 2 shows the position of psbA of lettuce to be base-edited;
FIG. 3 shows results confirming efficiency of adenine base editing after obtaining callus through culture following mRNA delivery;
FIG. 4 shows results confirming efficiency of adenine base editing 7 days after delivery of *in vitro-*synthesized mRNA to lettuce protoplasts;
FIG. 5 shows results confirming efficiency of adenine base editing after obtaining callus through culture following mRNA delivery; and
FIG. 6 shows results of psbA DNA sequence alignment of various plants.

### [Mode for Invention]

Unless otherwise defined, all technical and scientific terms used herein have the same meanings as typically understood by those skilled in the art to which the present invention belongs. In general, the nomenclature used herein is well known in the art and is typical.

The only method of editing the base of plant cell organelle DNA was to edit cytosine to thymine by attaching DddAtox to TALE. The technical task according to the present invention is intended to base-edit adenine to guanine in cell organelle DNA by attaching DddAtox and an adenine deaminase to TALE. By base-editing adenine of the cell organelle DNA, which was conventionally impossible, it is possible to develop functional plants, such as plants with herbicide resistance, etc.

The inventors of the present application manufactured a base editor capable of editing the base A by linking a DddAtox cytosine deaminase and an adenine deaminase capable of causing A to G conversion to a TALE or ZFP protein capable of binding to DNA.

A conventional deaminase (DdCBE) utilizing DddAtox is a cytosine deaminase that uses a TALE repeat as a DNA binding module. Unlike DdCBE, which only causes C to T conversion, DdABE may cause A to G conversion, creating different variation patterns.

DdABE recognizes double-stranded DNA by itself and causes deamination, and thus has no additional component such as RNA. For mitochondria or chloroplasts, the RNA delivery mechanism was not known, so the CRISPR system could not be applied. However, DdABE without such a component may not only target genomic DNA in cells, but also target DNA in organelles such as mitochondria or chloroplasts, causing A to G conversion in specific DNA.

DdABE is highly compatible because it may be employed using split DddAtox or a full-length DddAtox variant, as necessary.

As used herein, the term "editing" may be used interchangeably with "proofreading" and refers to a method of altering a nucleic acid sequence by selective deletion of a specific genomic target. The specific genomic target includes, but is not limited to, a chromosomal region, a gene, a promoter, an open reading frame, or any nucleic acid sequence.

As used herein, the "single base" refers to one, and only one, nucleotide within a nucleic acid sequence. In the context of single-base editing, this means that the base at a specific position within the nucleic acid sequence is replaced with a different base. This replacement may occur by many mechanisms, including but not limited to, substitution or modification.

As used herein, the term "target" or "target site" refers to a pre-identified nucleic acid sequence of any composition and/or length. The target site includes, but is not limited to, a chromosomal region, a gene, a promoter, an open reading frame, or any nucleic acid sequence.

As used herein, the term "on-target" refers to a subsequence of a specific genomic target that may be completely complementary to a programmable DNA binding domain and/or a single guide RNA sequence.

As used herein, the term "off-target" refers to a subsequence of a specific genomic target that may be partially complementary to a programmable DNA binding domain and/or a single guide RNA sequence.

The present invention includes a first split and a second split derived from a cytosine deaminase or variants thereof, in which each of the first split and the second split binds to the DNA binding protein.

The cytosine deaminase is an enzyme that removes an amino group and enables cytosine (C) to uridine (U) conversion.

The cytosine deaminase may be used. For example, there are types of cytosine deaminase, such as APOBEC1 (apolipoprotein B editing complex 1) and AID (activation-induced deaminase), but most DNA deaminases only work on single-stranded DNA and may not be suitable for base editing by linkage to a DNA binding protein. Specifically, the cytosine deaminase may be derived from a deaminase (DddA) acting on double-stranded DNA or an orthologue thereof. More specifically, the cytosine deaminase may be a double-stranded DNA-specific bacterial cytosine deaminase.

The cytosine deaminase is provided in split form, the cytosine deaminase includes a first split and a second split, and each of the first split and the second split has no deaminase activity.

The cytosine deaminase may include the sequence of SEQ ID NO: 1 corresponding to the tox split in a full-length cytosine deaminase. The cytosine deaminase includes a first split and a second split, and each of the first split and the second split has no deaminase activity.

[SEQ ID NO: 1]

GSYALGPYQISAPQLPAYNGQTVGTFYYVNDAGGLESKVFSSGGPTPY

PNYANAGHVEGQSALFMRDNGISEGLVFHNNPEGTCGFCVNMTETLLPENAKMTVV

PPEGAIPVKRGATGETKVFTGNSNSPKSPTKGGC

In one embodiment, the first split or the second split of the cytosine deaminase may include a sequence from the N-terminus to at least one selected from the group consisting of G33, G44, A54, N68, G82, N98, G108 in the sequence of SEQ ID NO: 1. The first split or the second split of the cytosine deaminase may include a sequence from at least one selected from the group consisting of G34, P45, G55, N69, T83, A99, and A109 to the C-terminus in the sequence of SEQ ID NO: 1.

Specifically, the cytosine deaminase may include a first split of SEQ ID NO: 2 (G1333-N) and a second split of SEQ ID NO: 3 (G1333-C), a first split of SEQ ID NO: 4 (G1397-N) and a second split of SEQ ID NO: 5 (G1397-C), a first split of SEQ ID NO: 2 (G1333-N) and a second split of SEQ ID NO: 5 (G1397-C), or a first split of SEQ ID NO: 4 (G1397-N) and a second split of SEQ ID NO: 2 (G1333-C).
(SEQ ID NO: 2) Wild-type DddAtox G1333-N 
GSYALGPYQISAPQLPAYNGQTVGTFYYVNDAGGLESKVFSSGG

GGCTCTGGTTCCTACGCCCTGGGTCCATATCAGATTAGTGCTCCCCAA

CTCCCCGCCTACAACGGTCAGACAGTGGGGACCTTTTACTATGTCAACGACGCCGG

GGGATTGGAATCCAAGGTTTTCTCTAGCGGTGGG
(SEQ ID NO: 3) Wild-type G1333-C 
PTPYPNYANAGHVEGQSALFMRDNGISEGLVFHNNPEGTCGFCVNMTE

TLLPENAKMTVVPPEGAIPVKRGATGETKVFTGNSNSPKSPTKGGC

CCAACACCTTATCCTAACTACGCTAACGCCGGGCACGTCGAGGGGCAG

TCAGCTCTTTTTATGAGAGATAACGGCATTAGCGAAGGGCTTGTGTTCCATAATAA

TCCTGAGGGCACCTGTGGCTTCTGTGTAAATATGACCGAAACACTTCTGCCTGAGA

ACGCTAAAATGACTGTCGTACCACCCGAAGGCGCAATCCCAGTTAAACGGGGCGCA

ACCGGCGAAACCAAAGTATTCACCGGAAACAGCAATAGTCCAAAGTCCCCCACCAA

GGGAGGTTGC
(SEQ ID NO: 4) Wild-type DddAtox G1397-N [53]    GSYALGPYQISAPQLPAYNGQTVGTFYYVNDAGGLESKVFSSGGPTPY 
PNYANAGHVEGQSALFMRDNGISEGLVFHNNPEGTCGFCVNMTETLLPENAKMTVV

PPEG

GGTAGCTACGCACTTGGTCCTTACCAGATTAGCGCACCCCAACTCCCC

GCCTATAATGGTCAAACCGTCGGACCTTTTACTACGTAAACGATGCTGGTGGGCT

GGAATCCAAAGTATTCTCCTCAGGGGGCCCTACACCCTACCCCAACTACGCCAATG

CTGGTCATGTAGAAGGGCAGTCAGCACTGTTTATGCGCGATAATGGTATAAGCGAG

GGGTTGGTCTTCCATAACAACCCAGAGGGTACTTGTGGCTTCTGTGTGAATATGAC

TGAAACCCTTCTGCCCGAAAATGCCAAGATGACTGTCGTCCCACCTGAAGGC
(SEQ ID NO: 5) Wild-type DddAtox G1397-C

AIPVKRGATGETKVFTGNSNSPKSPTKGGC

GCCATACCTGTGAAGCGGGGAGCAACAGGGGAGACAAAGGTGTTCACA

GGCAACTCTAACAGTCCAAAGAGCCCCACCAAAGGCGGGTGT

G1333N, G1333C, G1397N, and G1397C in combination may be used as a deaminase in split form. Specifically, there may be provided in the form of Left-G1333-N + Right-G1333-C, Left-G1397-N + Right-G1397-C, Left-G1397-N + Right-G1333-C, or Left-G1333-N + Right-G1397-C.

The DNA binding protein may be, for example, a zinc finger protein, a TALE (transcription activator-like effector) array, or a combination thereof.

The zinc finger motif of the zinc finger protein has a DNA binding domain, and the c-terminal portion of the finger specifically recognizes a DNA sequence. DNA binding proteins including 3-6 zinc finger motifs recognize DNA sequences.

In one embodiment, each of the first split of the cytosine deaminase and the second split of the cytosine deaminase may bind to the N-terminus or C-terminus of the zinc finger protein.

Binding of the C-terminus of the zinc finger protein (ZF-Left) to the N-terminus of the first split of the cytosine deaminase, and binding of the C-terminus of the zinc finger protein (ZF-Right) to the N-terminus of the second split of the cytosine deaminase (CC configuration);

Binding of the N-terminus of the zinc finger protein (ZF-Left) to the C-terminus of the first split of the cytosine deaminase, and binding of the C-terminus of the zinc finger protein (ZF-Right) to the N-terminus of the second split of the cytosine deaminase (NC configuration);

Binding of the C-terminus of the zinc finger protein (ZF-Left) to the N-terminus of the first split of the cytosine deaminase, and binding of the N-terminus of the zinc finger protein (ZF-Right) to the C-terminus of the second split of the cytosine deaminase (CN configuration); or

Binding of the N-terminus of the zinc finger protein (ZF-Left) to the C-terminus of the first split of the cytosine deaminase, and binding of the N-terminus of the zinc finger protein (ZF-Right) to the C-terminus of the second split of the cytosine deaminase (NN configuration).

The ZF-Left may include the following sequence:

GIHGVPAAMAERPFQCRICMRNFSDRSNLSRHIRTHTGEKPFACDICG
RKFAISSNLNSHTKIHTGSQKPFQCRICMRNFSRSDNLARHIRTHTGEKPFACDIC
GRKFATSGNLTRHTKIHLR.

The ZF-Right may include the following sequence:

GIHGVPAAMAERPFQCRICMRNFSRSDNLSVHIRTHTGEKPFACDICG
RKFAQKINLQVHTKIHTGEKPFQCRICMRNFSRSDVLSEHIRTHTGEKPFACDICG
RKFAQRNHRTTHTKIHLR.

The sequence of ZF may vary depending on the DNA target. ZF may be customized depending on the DNA target sequence. Since ZF recognizes 3 bp of DNA, it is possible to construct a ZF combination that recognizes 9-18 bp of DNA by connecting 3-6 ZFs. For example, construction is possible using a library that includes modules such as GNN, TNN, CNN, or ANN.

In some cases, the zinc finger protein may be linked to the deaminase via a linker. The linker may be a peptide linker including 2 to 40 amino acid residues. The linker may be, for example, a linker having a length of 2aa, 5aa, 10aa, 16aa, 24aa, or 32aa, but is not limited thereto.

In one embodiment, the linker may include:
2a.a linker: GS
5a.a linker: TGEKQ
10a.a linker: SGAQGSTLDF
16a.a linker: SGSETPGTSESATPES
24 a.a linker: SGTPHEVGVYTLSGTPHEVGVYTL or
32a.a linker: GSGGSSGGSSGSETPGTSESATPESSGGSSGGS.

In a specific embodiment according to the present invention, the split deaminase and the zinc finger protein may be linked via a linker, the zinc finger protein binds to the N-terminus of the half deaminase including the first split, and the zinc finger protein binds to the N-terminus of the half deaminase including the second split. As such, C to T base conversion may occur in the spacer between sites to which the left and right ZFPs are attached. Both the left and right ZFPs were confirmed to exhibit high editing efficiency when linked respectively to the half deaminase including the first split and the half deaminase including the second split via a 24 a.a linker.

The TAL effector (TALE) is composed of repeats, each 33-34 amino acids long, with about 9 domains repeated (repeated variant domains (RVDs)). The TAL effector may recognize one nucleotide per domain and may bind to a specific DNA sequence depending on the 12^{th}-13^{th} amino acid sequence (HD->cytosine, NI->adenine, NG->thymine, NN->guanine). The TAL effector (TALE) recognizes a single DNA strand within the target site. The distance between target sites may be 12-14 nucleotides.

The TALE domain indicates a protein domain that binds to a nucleotide in a sequence-specific manner by combination of at least one TALE-repeat unit. The TALE domain includes at least one TALE-repeat unit, particularly 1 to 30 TALE-repeat units, but is not limited thereto. TALE-repeat is a site that recognizes a specific nucleotide sequence in the TALE domain.

The TALE domain includes a region containing the N-terminus of TALE and a region containing the C-terminus of TALE as a backbone structure.

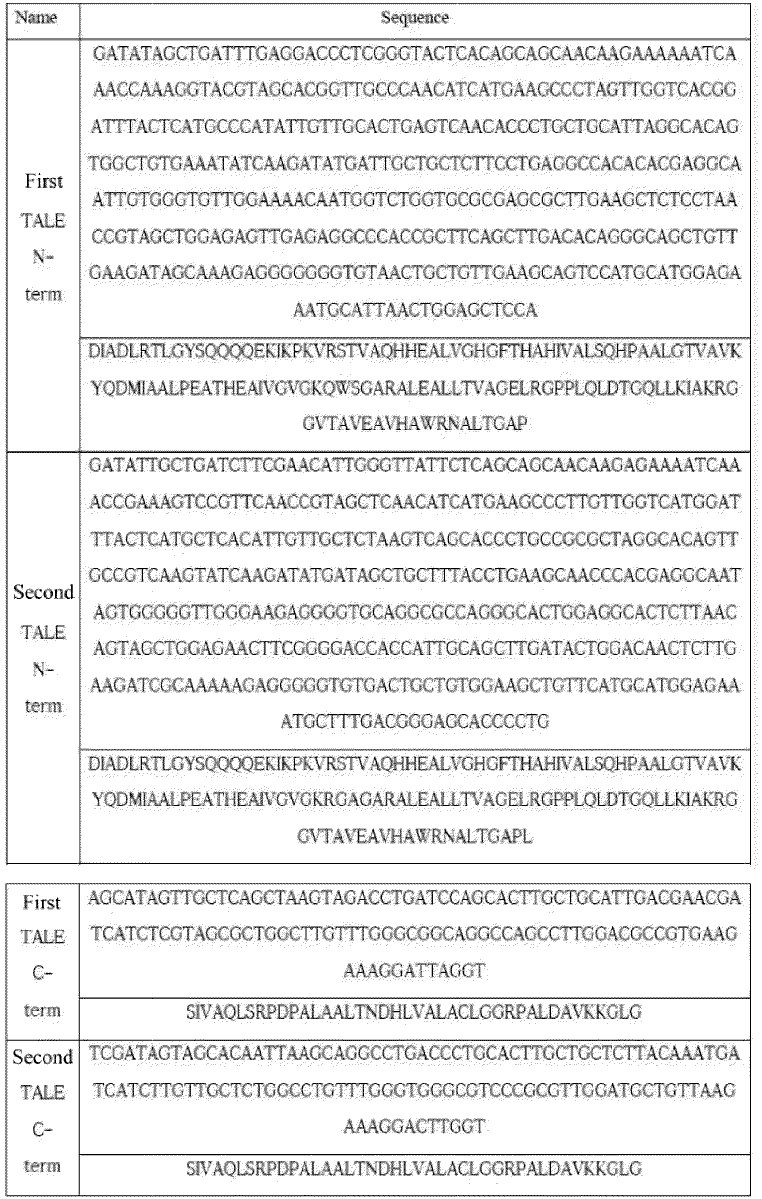

Depending on the binding position of the TALE domain based on the cleavage site, a single TALE array or a first TALE array and a second TALE array may bind.

A first TALE (left TALE) may bind to the first split of the cytosine deaminase, and a second TALE (right TALE) may bind to the second split of the cytosine deaminase, forming respective structures of N'-TALE-first split-C' and N'-TALE-second split-C'.

The TALE array may be customized depending on the target DNA sequence. The TALE array is configured such that modules composed of 33-35 amino acid residues are repeatedly arranged. TALEs are derived from the plant pathogen Xanthomonas, and each module recognizes each of A, C, G, and T bases and binds to DNA. The base specificity of each module is determined by the 12^{th} and 13^{th} amino acid residues, which are called repeat variable di-residue (RVD). For example, a module where RVD is NN recognizes G, NI recognizes A, HD recognizes C, and NG recognizes T. The TALE array is composed of at least 14 to 18 modules and may be designed to recognize 15-20 bp of a target DNA sequence.

A Cas protein may be included in mutated form, which may mean that it is mutated to eliminate endonuclease activity that cleaves double-stranded DNA, and there is exemplified at least one selected from among mutation target-specific nucleases mutated to eliminate endonuclease activity and have nickase activity and forms mutated to eliminate both endonuclease activity and nickase activity.

When having nickase activity, simultaneously with base conversion by the cytosine deaminase (e.g., cytosine to uridine conversion) or sequentially regardless of the order, a nick may be introduced to the strand where the base conversion occurs or the opposite strand (e.g. the strand opposite the strand where base conversion occurs) (e.g. a nick is introduced at a position between the 3^{rd} nucleotide and the 4^{th} nucleotide in a direction of the 5' end of the PAM sequence on the strand opposite the strand where PAM is located). Such mutations (e.g. amino acid substitutions, etc.) may occur in a catalytically active domain (e.g. a RuvC catalytic domain in Cas9). Also, *Streptococcus pyogenes*-derived Cas9 may include mutations in which at least one selected from the group consisting of a catalytic aspartate residue (aspartic acid at position 10 (D10), etc.), glutamic acid at position 762 (E762), histidine at position 840 (H840), asparagine at position 854 (N854), asparagine at position 863 (N863), aspartic acid at position 986 (D986), and the like is substituted with any different amino acid. Here, the different amino acid that is substituted may be alanine, but is not limited thereto.

In some cases, a *Streptococcus pyogenes*-derived Cas9 protein may be mutated to recognize NGA (in which N is any base selected from among A, T, G, and C) that is different from the PAM sequence (NGG) of wild-type Cas9 by substituting one or more selected from among aspartic acid at position 1135 (D1135), arginine at position 1335 (R1335), and threonine at position 1337 (T1337), for example, all three, with different amino acids.

For example, in the amino acid sequences of the *Streptococcus pyogenes*-derived Cas9 protein, amino acid substitution may occur at:
(1) D10, H840, or D10 + H840;
(2) D1135, R1335, T1337, or D1135 + R1335 + T1337; or
(3) both residues (1) and (2).

Here, the "different amino acid" may be alanine, isoleucine, leucine, methionine, phenylalanine, proline, tryptophan, valine, asparagine, cysteine, glutamine, glycine, serine, threonine, tyrosine, aspartic acid, glutamic acid, arginine, histidine, or lysine, and may refer to an amino acid selected from among amino acids excluding amino acids at original mutation positions in a wild-type protein from all known variants of the amino acids described above. For example, the "different amino acid" may be alanine, valine, glutamine, or arginine.

In some cases, guide RNA may be further included. Here, guide RNA may be at least one selected from the group consisting of, for example, CRISPR RNA (crRNA), trans-activating crRNA (tracrRNA), and single guide RNA (sgRNA). Specifically, guide RNA may be a double-stranded crRNA:tracrRNA complex in which crRNA and tracrRNA are linked to each other, or a single-stranded guide RNA (sgRNA) in which crRNA or a portion thereof and tracrRNA or a portion thereof are linked via an oligonucleotide linker.

The present invention includes an adenine deaminase. The adenine deaminase may be selected from the group consisting of, for example, APOBEC1 (apolipoprotein B editing complex 1), AID (activation-induced deaminase), and tadA (tRNA-specific adenosine deaminase), and specifically may be tadA (tRNA-specific adenosine deaminase). The adenine deaminase may be, for example, a deoxy-adenine deaminase as a variant of *E. coli* TadA.

In a structure in which the cytosine deaminase is included in split form; the DNA binding protein is a zinc finger protein; the N-terminus of the zinc finger protein (ZF-Left) binds to the C-terminus of the first split of the cytosine deaminase; and the C-terminus of the zinc finger protein (ZF-Right) binds to the N-terminus of the second split of the cytosine deaminase (NC configuration), the adenine deaminase may bind to the C-terminus of the zinc finger protein (ZF-Left), the N-terminus or C-terminus of the first split of the cytosine deaminase, the N-terminus of the zinc finger protein (ZF-Right), or the N-terminus or C-terminus of the second split of the cytosine deaminase.

The adenine deaminase may bind to the C-terminus of the zinc finger protein (ZF-Left), the N-terminus or C-terminus of the first split of the cytosine deaminase, the N-terminus of the zinc finger protein (ZF-Right), or the N-terminus or C-terminus of the second split of the cytosine deaminase even in structures in which the C-terminus of the zinc finger protein (ZF-Left) binds to the N-terminus of the first split of the cytosine deaminase and the C-terminus of the zinc finger protein (ZF-Right) binds to the N-terminus of the second split of the cytosine deaminase (CC configuration); the C-terminus of the zinc finger protein (ZF-Left) binds to the N-terminus of the first split of the cytosine deaminase and the N-terminus of the zinc finger protein (ZF-Right) binds to the C-terminus of the second split of the cytosine deaminase (CN configuration); or the N-terminus of the zinc finger protein (ZF-Left) binds to the C-terminus of the first split of the cytosine deaminase and the N-terminus of the zinc finger protein (ZF-Right) binds to the C-terminus of the second split of the cytosine deaminase (NN configuration).

When the cytosine deaminase is included in split form and the DNA binding protein is TALE, a first TALE may bind to the first split of the cytosine deaminase and a second TALE may bind to the second split of the cytosine deaminase, forming respective structures of N'-TALE-first split DDDA-C' and N'-TALE-second split DDDA-C'. The adenine deaminase may bind to the N-terminus or C-terminus of the first split of the cytosine deaminase or to the N-terminus or C-terminus of the second split of the cytosine deaminase.

The present invention relates to a composition (UGI-free) for A-to-G base editing in plant cell organelle DNA, including 1) a DNA binding protein, 2) a split double-stranded DNA-specific bacterial cytosine deaminase, and 3) a deoxy-adenine deaminase derived from *E. coli* TadA, in which the DNA binding protein is a zinc finger protein (ZFP) or a transcription activator-like effector (TALE) array, and the split double-stranded DNA-specific bacterial cytosine deaminase is DddAtox derived from *Burkholderia cenocepacia.*

In a structure in which the cytosine deaminase is included in split form; the DNA binding protein is a zinc finger protein; the N-terminus of the zinc finger protein (ZF-Left) binds to the C-terminus of the first split of the cytosine deaminase; and the C-terminus of the zinc finger protein (ZF-Right) binds to the N-terminus of the second split of the cytosine deaminase (NC configuration), the adenine deaminase may bind to the C-terminus of the zinc finger protein (ZF-Left), the N-terminus or C-terminus of the first split of the cytosine deaminase, the N-terminus of the zinc finger protein (ZF-Right), or the N-terminus or C-terminus of the second split of the cytosine deaminase.

The adenine deaminase may bind to the C-terminus of the zinc finger protein (ZF-Left), the N-terminus or C-terminus of the first split of the cytosine deaminase, the N-terminus of the zinc finger protein (ZF-Right), or the N-terminus or C-terminus of the second split of the cytosine deaminase even in structures in which the C-terminus of the zinc finger protein (ZF-Left) binds to the N-terminus of the first split of the cytosine deaminase, and the C-terminus of the zinc finger protein (ZF-Right) binds to the N-terminus of the second split of the cytosine deaminase (CC configuration); the C-terminus of the zinc finger protein (ZF-Left) binds to the N-terminus of the first split of the cytosine deaminase, and the N-terminus of the zinc finger protein (ZF-Right) binds to the C-terminus of the second split of the cytosine deaminase (CN configuration); or the N-terminus of the zinc finger protein (ZF-Left) binds to the C-terminus of the first split of the cytosine deaminase, and the N-terminus of the zinc finger protein (ZF-Right) binds to the C-terminus of the second split of the cytosine deaminase (NN configuration).

When the cytosine deaminase is included in split form and the DNA binding protein is TALE, a first TALE may bind to the first split of the cytosine deaminase and a second TALE may bind to the second split of the cytosine deaminase, forming respective structures of N'-TALE-first split DDDA-C' and N'-TALE-second split DDDA-C'. The adenine deaminase may bind to the N-terminus or C-terminus of the first split of the cytosine deaminase or to the N-terminus or C-terminus of the second split of the cytosine deaminase.

The present invention relates to a composition for A-to-G base editing in a plant cell organelle, in which the DNA binding protein is a zinc finger protein or a TALE array, and the first and second splits derived from the cytosine deaminase are of bacterial origin and are specific for double-stranded DNA.

The present invention relates to a composition for A-to-G base editing in a plant cell organelle, in which the DNA binding protein is a zinc finger protein or a TALE array, the first and second splits derived from the cytosine deaminase are of bacterial origin and are specific for double-stranded DNA, the DNA binding protein is attached to the N-terminus of the first split, and the DNA binding protein is attached to the C-terminus of the adenine deaminase.

The present invention relates to a composition for A-to-G base editing in a plant cell organelle, including 1) a DNA binding protein, 2) a split double-stranded DNA-specific bacterial cytosine deaminase, and 3) a deoxy-adenine deaminase derived from *E. coli* TadA, in which the composition is UGI-free, the DNA binding protein is a zinc finger protein (ZFP) or a TALE array, and the split double-stranded DNA-specific bacterial cytosine deaminase is DddAtox derived from *Burkholderia cenocepacia.*

The present invention relates to a method for A-to-G base editing in a plant cell organelle, including treating a plant cell with a DNA binding protein or a nucleic acid encoding the same, a first split and a second split derived from a cytosine deaminase or a nucleic acid encoding the same, and an adenine deaminase or a nucleic acid encoding the same, in which the DNA binding protein is a zinc finger protein or a TALE array, and the first split and the second split derived from the cytosine deaminase are of bacterial origin and are specific for double-stranded DNA.

The present invention relates to a method for A-to-G base editing in a plant cell organelle, including treating a plant cell with a DNA binding protein or a nucleic acid encoding the same, a first split and a second split derived from a cytosine deaminase or a nucleic acid encoding the same, and an adenine deaminase or a nucleic acid encoding the same, in which the DNA binding protein is a zinc finger protein or a TALE array, the first split and the second split derived from the cytosine deaminase are of bacterial origin and are specific for double-stranded DNA, the DNA binding protein is attached to the N-terminus of the first split, and the DNA binding protein is attached to the C-terminus of the adenine deaminase.

The present invention relates to a method for A-to-G base editing in a plant cell organelle, including treating a plant cell with a DNA binding protein or a nucleic acid encoding the same, a first split and a second split derived from a cytosine deaminase or a nucleic acid encoding the same, and an adenine deaminase or a nucleic acid encoding the same, particularly with a composition for A-to-G base editing in a plant cell organelle including 1) a DNA binding protein, 2) a split double-stranded DNA-specific bacterial cytosine deaminase, and 3) a deoxy-adenine deaminase derived from *E. coli* TadA, in which the composition is UGI-free, the DNA binding protein is a zinc finger protein (ZFP) or a TALE array, and the split double-stranded DNA-specific bacterial cytosine deaminase is DddAtox derived from *Burkholderia cenocepacia.*

The present invention may further include a chloroplast transit peptide or a mitochondrial targeting signal (MTS).

For example, the chloroplast transit peptide (CTP) or mitochondrial targeting signal (MTS) binds and is delivered to chloroplasts and mitochondria in plant cells. When delivered to the chloroplasts and mitochondria, the remainder except for the N-terminal CTP or MTS protein is delivered in the form of preprotein into the chloroplasts and mitochondria. During the process of entering the chloroplasts and mitochondria, the transit protein moiety is detached, and a specific portion may be base-edited by targeting the chloroplasts and mitochondria.

Regarding the nucleic acid, "polynucleotide", "nucleotide", "nucleotide sequence", and "oligonucleotide" are used interchangeably. Polymeric forms of nucleotides of any length, deoxyribonucleotides or ribonucleotides, or analogues thereof may be included. Polynucleotides may have any three-dimensional structure and may perform any function, known or unknown. A polynucleotide may include one or more modified nucleotides, such as methylated nucleotides and nucleotide analogues. Modification to the nucleotide structure is possible before or after assembly of the polymer.

The polynucleotide may be an RNA sequence, a DNA sequence, or a combination thereof (RNA-DNA hybrid sequence).

As a means for expressing the fusion protein, known expression vectors such as plasmid vectors, cosmid vectors, bacteriophage vectors, etc. may be used, and vectors may be easily prepared by those skilled in the art according to any known method using recombinant DNA technology.

The vector may be a plasmid vector or a viral vector, and specific examples of the viral vector may include, but are not limited to, adenovirus, adeno-associated virus, lentivirus, and retrovirus vectors.

A recombinant expression vector may contain a nucleic acid in a form suitable for expression of the nucleic acid in a host cell, which means that it contains at least one regulatory element that may be selected on a host cell basis so that the recombinant expression vector is used for expression, namely is operably linked to the nucleic acid sequence to be expressed.

Within the recombinant expression vector, "operably linked" means that the nucleotide sequence of interest is connected to the regulatory element in a manner that allows expression of the nucleotide sequence (e.g., in an *in vitro* transcription/translation system or in a host cell when the vector is introduced into the host cell).

The recombinant expression vector may include forms suitable for messenger RNA synthesis, including a T7 promoter, which means that it includes at least one regulatory element to enable *in situ* mRNA synthesis, namely messenger RNA synthesis by T7 polymerase.

The "regulatory element" may include a promoter, an enhancer, an internal ribosome entry site (IRES), and other expression control elements (e.g., transcription termination signals such as polyadenylation signals and poly-U sequences). The regulatory element includes elements that direct the induction or constitutive expression of a nucleotide sequence in many types of host cells, and elements that direct expression of a nucleotide sequence only in certain host cells (e.g., tissue-specific regulatory sequences). A tissue-specific promoter may direct expression primarily in a desired tissue of interest such as muscle, neuron, bone, skin, blood, a specific organ (e.g., liver, pancreas), or a specific cell type (e.g. lymphocyte). The regulatory element may also direct expression in a temporally-dependent manner, such as in a cell cycle-dependent or developmental stage-dependent manner, which may or may not be tissue- or cell-type specific.

In some cases, the vector includes at least one pol III promoter, at least one pol II promoter, at least one pol I promoter, or combinations thereof. Examples of the pol III promoter include, but are not limited to, U6 and H1 promoters. Examples of the pol II promoter include, but are not limited to, retroviral Rous sarcoma virus (RSV) LTR promoter (optionally with RSV enhancer), cytomegalovirus (CMV) promoter (optionally with CMV enhancer) (e.g., Boshart et al. (1985) Cell 41:521-530), SV40 promoter, dihydrofolate reductase promoter, β-actin promoter, phosphoglycerol kinase (PGK) promoter, and EF1α promoter.

The "regulatory element" includes enhancers such as WPRE; CMV enhancer; R-U5' segment in LTR of HTLV-I; SV40 enhancer; and an intronic sequence between exons 2 and 3 of rabbit β-globin. It will be appreciated by those skilled in the art that the design of the expression vector may depend on factors such as the choice of host cell to be transformed, the level of expression desired, and the like. The vector may be introduced into a host cell to produce a transcript, protein, or peptide including a fusion protein or peptide encoded by a nucleic acid as described herein (e.g., a clustered regularly interspaced short palindromic repeat (CRISPR) transcript, protein, enzyme, mutants thereof, fusion proteins thereof, etc.). Advantageous vectors include lentiviruses and adeno-associated viruses, and these types of vectors may also be selected to target a certain type of cell.

The vector may be delivered *in vivo* or into cells through local injection (e.g., direct injection into a lesion or target site), electroporation, lipofection, viral vector, nanoparticles, PTD (protein translocation domain) fusion protein method, etc.

The nucleic acid may be injected in the form of ribonucleic acid such as messenger ribonucleic acid mRNA, enabling gene base editing in cells, for example, in animal cells or plant cells, without limitation.

The nucleic acid according to the present invention may be in the form of mRNA, and when delivered in the form of mRNA, compared to delivery in the form of a vector using DNA, a transcription process into mRNA is unnecessary, so that gene editing may be initiated quickly. There is a high possibility of transient protein expression.

The inventors of the present application have ascertained that, when a cytosine base editor is introduced into a plant cell in the form of ribonucleic acid such as messenger ribonucleic acid, for the purpose of editing plant cell organelle genes, the off-target effect is reduced compared to when it is delivered by a plasmid. It was demonstrated for the first time that, when a cytosine base editor is transformed into plant cells in the form of mRNA, it has an advantage in off-target effects compared to plasmids in the editing of plant cell organelle genes.

The mRNA may be delivered directly or through a carrier. In some cases, the mRNA of the nuclease and/or the cleavage factor may be chemically modified or directly delivered in the form of synthetic self-replicative RNA.

Methods of delivering mRNA molecules to cells *in vitro* or *in vivo* may be contemplated, including methods of delivering mRNA to cells or methods of delivering mRNA to cells of organisms such as humans or animals *in vivo.* For example, mRNA molecules may be delivered to cells, including lipids (e.g., liposomes, micelles, etc.), nanoparticles or nanotubes, cationic compounds (e.g., polyethyleneimine or PEI), or cationic compounds (e.g., polyethyleneimine or PEI). In some cases, biolistics, a method using a gene gun or a biolistic particle delivery system, may be performed to deliver mRNA to cells.

Examples of the carrier may include, but are not limited to, a cell penetrating peptide (CPP), nanoparticles, and a polymer.

The CPP is a short peptide that facilitates cellular uptake of various molecular cargoes (from nanoscale particles to small chemical molecules and large fragments of DNA).

Regarding the nanoparticles, the composition according to the present invention may be delivered via polymer nanoparticles, metal nanoparticles, metal/inorganic nanoparticles, or lipid nanoparticles. The polymer nanoparticles may be, for example, DNA nanoclews, yarn-like DNA nanoparticles that are synthesized by rolling circle amplification. DNA nanoclews, yarn-like DNA nanoparticles, may be loaded with mRNA and coated with PEI to improve endosomal escape capacity. These complexes bind to cell membranes, are internalized, and then translocate to the nucleus through endosome escape, where they may be delivered.

Regarding the metal nanoparticles, gold particles may be connected and complexed with a cationic endosomal disruptive polymer and thus delivered to cells. Examples of the cationic endosomal disruptive polymer may include polyethyleneimine, poly(arginine), poly(lysine), poly(histidine), poly-[2-{(2-aminoethyl)amino}-ethyl-aspartamide] (pAsp(DET)), block copolymer of poly(ethylene glycol) (PEG) and poly(arginine), block copolymer of PEG and poly(lysine), and block copolymer of PEG and poly{N-[N-(2-aminoethyl)-2-aminoethyl]aspartamide} (PEG-pAsp(DET)).

Regarding the metal/inorganic nanoparticles, mRNA may be encapsulated through, for example, ZIF-8 (zeolitic imidazolate framework-8).

In some cases, the mRNA may be negatively charged and coupled with cationic materials to form nanoparticles, which may penetrate cells through receptor-mediated endocytosis or phagocytosis.

Examples of the cationic polymer may include polyallylamine (PAH); polyethyleneimine (PEI); poly(L-lysine) (PLL); poly(L-arginine) (PLA); polyvinylamine homopolymers or copolymers; poly(vinylbenzyl-tri-C1-C4-alkylammonium salts); polymers of aliphatic or alicyclic dihalides and aliphatic N,N,N',N'-tetra-C1-C4-alkyl-alkylenediamines; poly(vinylpyridine) or poly(vinylpyridinium salt); poly(N,N-diallyl-N,N-di-C1-C4-alkyl-ammonium halide); homopolymers or copolymers of quaternized di-C1-C4-alkyl-aminoethyl acrylates or methacrylates; POLYQUAD^{™}; polyaminoamide, and the like.

The cationic lipids may include cationic liposomal formulations. The lipid bilayer of liposomes may protect encapsulated nucleic acids from degradation and may prevent specific neutralization by antibodies capable of binding to nucleic acids. During endosomal maturation, endosome membranes and liposomes are fused, enabling efficient endosome escape of cationic lipid-nucleases. Examples of the cationic lipids may include polyethyleneimine, starburst polyamidoamine (PAMAM) dendrimers, Lipofectin (combination of DOTMA and DOPE), lipofectase, LIPOFECTAMINE^{®} (e.g., Lipofectamine^{®} 2000, Lipofectamine^{®} 3000, Lipofectamine^{®} RNAiMAX, Lipofectamine^{®} LTX), SAINT-RED (Synvolux Therapeutics, Groningen, Netherlands), DOPE, Cytopectin (Gilead Sciences, Foster City, California), and Eupectin (JBL, San Luis Obispo, California). Representative cationic liposomes may be prepared from N-[1-(2,3-dioleyloxy)propyl]-N,N,N-trimethylammonium chloride (DOTMA), N-[1-(2,3-dioleyloxy)propyl]-N,N,N-trimethylammonium methylsulfate (DOTAP), 3β-[N-(N',N' - dimethylaminoethane)carbamoyl]cholesterol (DC-Chol), 2,3-dioleyloxy-N-[2-(sperminecarboxamido)ethyl]-N,N-dimethyl-1-propanaminium trifluoroacetate (DOSPA), 1,2-dimyristyloxypropyl-3-dimethyl-hydroxyethyl ammonium bromide, or dimethyldioctadecylammonium bromide (DDAB).

Regarding the lipid nanoparticles, delivery is possible using liposomes as carriers. Liposomes are spherical vesicular structures composed of a unilamellar or multilamellar lipid bilayer surrounding an inner aqueous compartment and a relatively impermeable outer lipophilic phospholipid bilayer. Liposomal formulations may primarily contain natural phospholipids and lipids such as 1,2-distearoyl-sn-glycero-3-phosphatidylcholine (DSPC), sphingomyelin, phosphatidylcholine, or monosialoganglioside. In some cases, cholesterol or 1,2-dioleoyl-sn-glycero-3-phosphoethanolamine (DOPE) may be added to the lipid membrane in order to resolve instability in plasma. The addition of cholesterol reduces the rapid release of encapsulated bioactive compounds into plasma or 1,2-dioleoyl-sn-glycero-3-phosphoethanolamine (DOPE) serves to increase stability.

The composition may be delivered to plant cells through:
gene gun bombardment;
PEG-mediated protoplast transfection;
protoplast transfection by electroporation; or
protoplast injection by microinjection.

The polynucleotide sequence encoding the fusion protein according to the present invention may be an RNA sequence, a DNA sequence, or a combination thereof (RNA-DNA hybrid sequence).

The nucleic acid may be delivered to plant cells through:
transformation using Agrobacterium, such as *Agrobacterium tumefaciens, Agrobacterium rhizogene,* etc.
   - binary vector,
   - viral vector: geminivirus, tobacco rattle virus (TRV), tomato mosaic virus (ToMV), foxtail mosaic virus (FoMV), barley yellow striate mosaic virus (BYSMV), Sonchus yellow net rhabdovirus (SYNV), etc.;
viral transfection;
gene gun bombardment;
PEG-mediated protoplast transfection;
protoplast transfection by electroporation; or
protoplast injection by microinjection.

Examples of the virus may include a viral vector: geminivirus, tobacco rattle virus (TRV), tomato mosaic virus (ToMV), foxtail mosaic virus (FoMV), barley yellow striate mosaic virus (BYSMV), Sonchus yellow net rhabdovirus (SYNV), etc.

The vector may be delivered into cells through local injection (e.g., direct injection into a lesion or target site), electroporation, lipofection, viral vector, nanoparticles, PTD (protein translocation domain) fusion protein method, etc.

The present invention relates to a method of editing adenine to guanine in plant cell organelle DNA, including treating a plant cell with the composition described above.

The present invention relates to an atrazine herbicide-resistant plant in which the adenine base of the chloroplast psbA gene including the GTTGAAAGC sequence is edited to guanine by the method described above.

The present invention relates to a spectinomycin-resistant plant in which the adenine base of 16s rRNA including the CGTCATCCTCA sequence is edited to guanine by the method described above.

The present invention relates to a plant having an albino phenotype in which the adenine base of the psaA gene including ATG is edited to guanine by the method described above.

By base-editing phenylalanine, the 295^{th} amino acid of psbA in chloroplast DNA, to serine, plants resistant to atrazine, an herbicide, may be developed. Spectinomycin-resistant plants and albino-type plants may be developed through adenine base editing of 16s rRNA and psaA, as well as psbA in chloroplasts.

The present invention relates to a plant in which the adenine base of a chloroplast RuBisCO (ribulose bisphosphate carboxylase) coding gene is edited to guanine by the method described above. Since base editing of the RuBisCO large unit involved in photosynthesis is possible, it is expected that plant production will increase by controlling photosynthetic efficiency.

The base sequences of chloroplasts have high homology among plants, so not only the lettuce (Lactuca sativa cv. Cheongchima) used in the present invention, but also plants such as rice, wheat, potatoes, tomatoes and the like may be developed to be resistant to atrazine.

### Examples

A better understanding of the present invention may be obtained through the following examples. These examples are merely set forth to illustrate the present invention and are not to be construed as limiting the scope of the present invention, as will be apparent to those skilled in the art.

### Example 1.

According to a paper published in Scientific Reports 8: 5179 (2018), it was reported that *E. coli* becomes resistant to spectinomycin by substituting uracil at positions 1183 and 1189 of 16s rRNA of *E. coli* with cytosine, either individually or simultaneously. When substituting adenine with guanine at positions 1183 and 1189 of 16s rRNA present in plant chloroplasts, it is possible to develop plants resistant to spectinomycin.

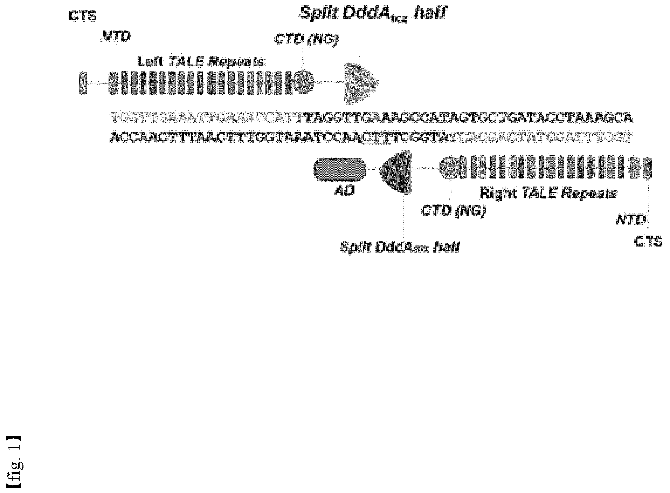

The sequence for each component of the adenine base editor used in Examples is as follows. FIG. 1 schematically shows the configuration in which DddAtox and adenine deaminase are attached to TALE.
CTS 
MDSQLVLSLKLNPSFTPLSPLFPFTPCSSFSPSLRFSSCYSRRLYSPV

TVYAAK
NTD 
DLRTLGYSQQQQEKIKPKVRSTVAQHHEALVGHGFTHAHIVALSQHPA
ALGTVAVKYQDMIAALPEATHEAIVGVGKQWSGARALEALLTVAGELRGPPLQLDT
GQLLKIAKRGGVTAVEAVHAWRNALTGAPLN
CTD 
LTPEQVVAIASNGGGKQALESIVAQLSRPDPALAALTNDHLVALACLG

GRPALDAVKKGLG
AD 
SEVEFSHEYWMRHALTLAKRARDEREVPVGAVLVLNNRVIGEGWNRAI
GLHDPTAHAEIMALRQGGLVMQNYRLIDATLYVTFEPCVMCAGAMIHSRIGRVVFG
VRNSKRGAAGSLMNVLNYPGMNHRVEITEGILADECAALLCDFYRMPRQVFNAQKK
AQSSIN
Linker 1 (TALE array - Linker 1-DddAₜₒₓ)
GS
Linker 2 (DddAtₒₓ-Linker 2-AD)
SGSETPGTSESATPES
DddAₜₒₓ G1347N 
GSGSYALGPYQISAPQLPAYNGQTVGTFYYVNDAGGLESKVFSSGGPT
PYPNYANAGHVEGQSALFMRDNGISEGLVFHNNPEGTCGFCVNMTETLLPENAKMT
VVPPEG
DddAₜₒₓ G1397C
GSAIPVKRGATGETKVFTGNSNSPKSPTKGGC
psbA Left TALE repeat 
LTPAQVVAIASNGGGKQALETVQRLLPVLCQAHGLTPAQVVAIASNNG
GKQALETVQRLLPVLCQDHGLTPAQVVAIASNIGGKQALETVQRLLPVLCQDHGLT
PDQVVAIASNIGGKQALETVQRLLPVLCQAHGLTPDQVVAIASNIGGKQALETVQR
LLPVLCQDHGLTPDQVVAIASNGGGKQALETVQRLLPVLCQAHGLTPAQVVAIASN
GGGKQALETVQRLLPVLCQAHGLTPAQVVAIASNNGGKQALETVQRLLPVLCQDHG
LTPAQVVAIASNIGGKQALETVQRLLPVLCQAHGLTPDQVVAIASNIGGKQALETV
QRLLPVLCQAHGLTPDQVVAIASNIGGKQALETVQRLLPVLCQAHGLTPAQVVAIA
SHDGGKQALETVQRLLPVLCQAHGLTPAQVVAIASHDGGKQALETVQRLLPVLCQD
HGLTPDQVVAIASNIGGKQALETVQRLLPVLCQDHGLTPAQVVAIAS
 
NGGGKQALETVQRLLPVLCQAHGLTPEQVVAIASNGGGKQALETVQRL
LPVLCQAHGLTPDQVVAIASNGGGKQALETVQRLLPVLCQAHG
psbA Right TALE repeat 
LTPDQVVAIASNNGGKQALETVQRLLPVLCQAHGLTPAQVVAIASHDG
GKQALETVQRLLPVLCQDHGLTPDQVVAIASNGGGKQALETVQRLLPVLCQAHGLT
PEQVVAIASNGGGKQALETVQRLLPVLCQAHGLTPDQVVAIASNGGGKQALETVQR
LLPVLCQDHGLTPDQVVAIASNIGGKQALETVQRLLPVLCQDHGLTPAQVVAIASN
NGGKQALETVQRLLPVLCQDHGLTPAQVVAIASNNGGKQALETVQRLLPVLCQDHG
LTPDQVVAIASNGGGKQALETVQRLLPVLCQAHGLTPDQVVAIASNIGGKQALETV
QRLLPVLCQDHGLTPAQVVAIASNGGGKQALETVQRLLPVLCQAHGLTPAQVVAIA
SHDGGKQALETVQRLLPVLCQDHGLTPAQVVAIASNIGGKQALETVQRLLPVLCQD
HGLTPDQVVAIASNNGGKQALETVQRLLPVLCQDHGLTPEQVVAIASHDGGKQALE
TVQRLLPVLCQAHGLTPEQVVAIASNIGGKQALETVQRLLPVLCQAHGLTPAQVVA
IASHDGGKQALETVQRLLPVLCQDHG
16s rRNA Left TALE repeat 
LTPAQVVAIASNIGGKQALETVQRLLPVLCQDHGLTPDQVVAIASNIG
GKQALETVQRLLPVLCQDHGLTPDQVVAIASNNGGKQALETVQRLLPVLCQAHGLT
PAQVVAIASNNGGKQALETVQRLLPVLCQAHGLTPDQVVAIASNNGGKQALETVQR
LLPVLCQDHGLTPEQVVAIASNNGGKQALETVQRLLPVLCQAHGLTPAQVVAIASH
DGGKQALETVQRLLPVLCQDHGLTPDQVVAIASNIGGKQALETVQRLLPVLCQDHG
LTPAQVVAIASNGGGKQALETVQRLLPVLCQAHGLTPAQVVAIASNNGGKQALETV
QRLLPVLCQDHGLTPAQVVAIASNIGGKQALETVQRLLPVLCQAHGLTPEQVVAIA
SNGGGKQALETVQRLLPVLCQAHGLTPAQVVAIASNNGGKQALETVQRLLPVLCQD
HGLTPAQVVAIASNIGGKQALETVQRLLPVLCQAHGLTPDQVVAIASHDGGKQALE
TVQRLLPVLCQAHGLTPEQVVAIASNGGGKQALETVQRLLPVLCQAHGLTPAQVVA
 
IASNGGGKQALETVQRLLPVLCQAHGLTPDQVVAIASNNGGKQALETVQRLLPVLC

QDHG
16s rRNA Right TALE repeat 
LTPDQVVAIASNNGGKQALETVQRLLPVLCQAHGLTPDQVVAIASHDG
GKQALETVQRLLPVLCQDHGLTPDQVVAIASHDGGKQALETVQRLLPVLCQAHGLT
PAQVVAIASNNGGKQALETVQRLLPVLCQDHGLTPAQVVAIASNNGGKQALETVQR
LLPVLCQDHGLTPDQVVAIASNGGGKQALETVQRLLPVLCQAHGLTPAQVVAIASN
NGGKQALETVQRLLPVLCQDHGLTPAQVVAIASNIGGKQALETVQRLLPVLCQAHG
LTPEQVVAIASNGGGKQALETVQRLLPVLCQAHGLTPAQVVAIASNIGGKQALETV
QRLLPVLCQDHGLTPD
 
QVVAIASNIGGKQALETVQRLLPVLCQDHGLTPDQVVAIASNNGGKQA
LETVQRLLPVLCQAHGLTPAQVVAIASHDGGKQALETVQRLLPVLCQDHGLTPDQV
VAIASHDGGKQALETVQRLLPVLCQAHGLTPEQVVAIASNNGGKQALETVQRLLPV
LCQAHGLTPDQVVAIASNNGGKQALETVQRLLPVLCQDHGLTPAQVVAIASNIGGK
QALETVQRLLPVLCQAHGLTPEQVVAIASNNGGKQALETVQRLLPVLCQAHG
psaA Left TALE repeat 
LTPAQVVAIASHDGGKQALETVQRLLPVLCQAHGLTPAQVVAIASNGG
GKQALETVQRLLPVLCQDHGLTPDQVVAIASNNGGKQALETVQRLLPVLCQDHGLT
PAQVVAIASNNGGKQALETVQRLLPVLCQDHGLTPEQVVAIASNGGGKQALETVQR
LLPVLCQAHGLTPAQVVAIASNGGGKQALETVQRLLPVLCQDHGLTPAQVVAIASH
DGGKQALETVQRLLPVLCQDHGLTPDQVVAIASHDGGKQALETVQRLLPVLCQAHG
LTPEQVVAIASNNGGKQALETVQRLLPVLCQAHGLTPAQVVAIASNNGGKQALETV
QRLLPVLCQAHGLTPDQVVAIASHDGGKQALETVQRLLPVLCQDHGLTPAQVVAIA
SNNGGKQALETVQRLLPVLCQAHGLTPDQVVAIASNIGGKQALETVQRLLPVLCQA
HGLTPDQVVAIASNIGGKQALETVQRLLPVLCQAHGLTPAQVVAIASHDGGKQALE
 
TVQRLLPVLCQAHGLTPEQVVAIASNNGGKQALETVQRLLPVLCQAHGLTPDQVVA
IASNIGGKQALETVQRLLPVLCQDHGLTPDQVVAIASNIGGKQALETVQRLLPVLC
QAHG
psaA Right TALE repeat

LTPEQVVAIASNNGGKQALETVQRLLPVLCQAHGLTPDQVVAIASNGG
GKQALETVQRLLPVLCQDHGLTPAQVVAIASNNGGKQALETVQRLLPVLCQAHGLT
PEQVVAIASNGGGKQALETVQRLLPVLCQAHGLTPAQVVAIASNGGGKQALETVQR
LLPVLCQAHGLTPDQVVAIASNNGGKQALETVQRLLPVLCQDHGLTPAQVVAIASN
GGGKQALETVQRLLPVLCQDHGLTPAQVVAIASHDGGKQALETVQRLLPVLCQAHG
LTPEQVVAIASHDGGKQALETVQRLLPVLCQAHGLTPAQVVAIASNNGGKQALETV
QRLLPVLCQAHGLTPDQVVAIASNNGGKQALETVQRLLPVLCQDHGLTPAQVVAIA
SNIGGKQALETVQRLLPVLCQAHGLTPAQVVAIASNIGGKQALETVQRLLPVLCQD
HGLTPDQVVAIASNIGGKQALETVQRLLPVLCQDHGLTPDQVVAIASNNGGKQALE
TVQRLLPVLCQAHGLTPAQVVAIASNIGGKQALETVQRLLPVLCQAHGLTPAQVVA
IASNNGGKQALETVQRLLPVLCQDHGLTPAQVVAIASNNGGKQALETVQRLLPVLC
QAHG

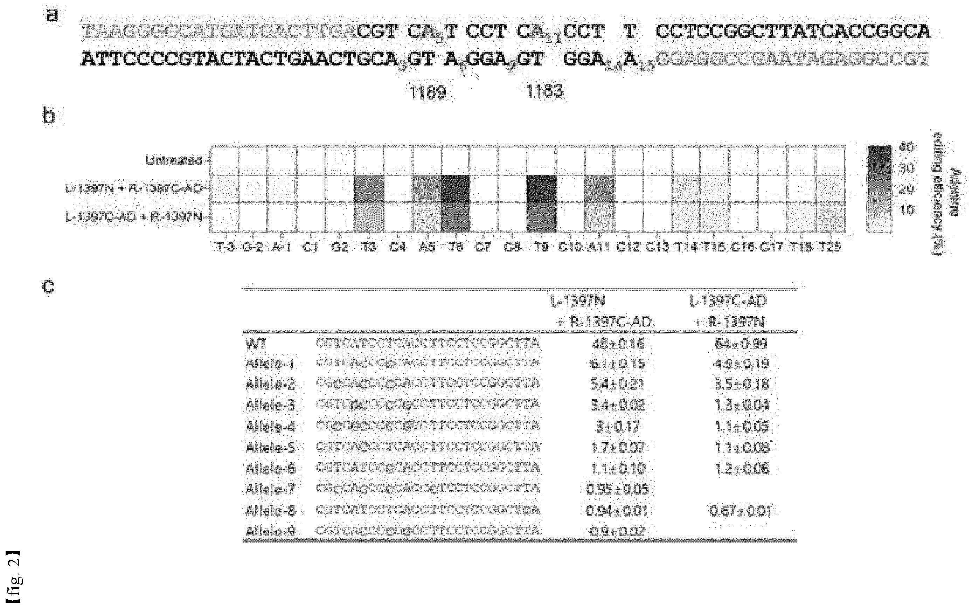

7 days after delivery of 30 µg of *in vitro-*synthesized mRNA to lettuce protoplasts, the efficiency of adenine base editing was confirmed, and the results thereof are shown in FIGs. 2a to 2c. FIG. 2a shows binding of the left and right TALE arrays to the DNA sequence in blue. A5 and A11, marked in red, are the target adenines of 16sRNA. According to FIG. 2b, the base editing rates of A5 are 17.27% and 6.91%, and those of A11 are 18.17% and 7.7%. In FIG. 2c, adenine targeting WT is marked in blue, and the alleles in which targets are changed are Allele-3, Allele-4, and Allele-9.

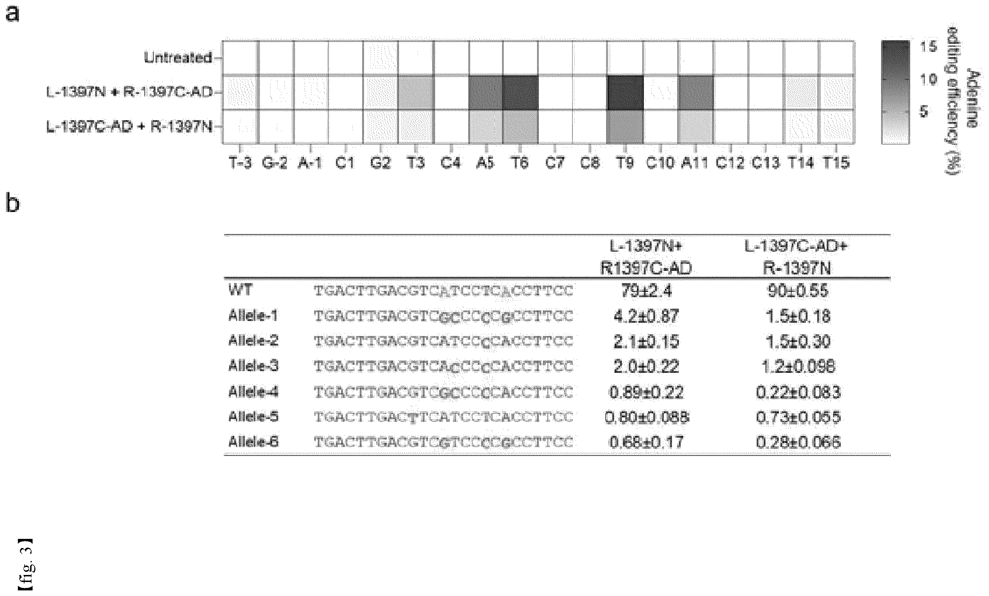

After callus was obtained through culture following mRNA delivery, the efficiency of adenine base editing was confirmed. According to FIG. 3a, the base editing rates of A5 are 9.7% and 2.9%, and those of A11 are 9.15% and 3.06%. According to FIG. 3b, adenine targeting WT is marked in blue, and the alleles in which targets are changed are Allele-1, Allele-4, and Allele-6.

### Example 2.

Mutations in psaA in chloroplasts result in plants with an albino phenotype. In the present invention, it is possible to develop a plant having an albino phenotype by disrupting ATG in the start codon of psaA.

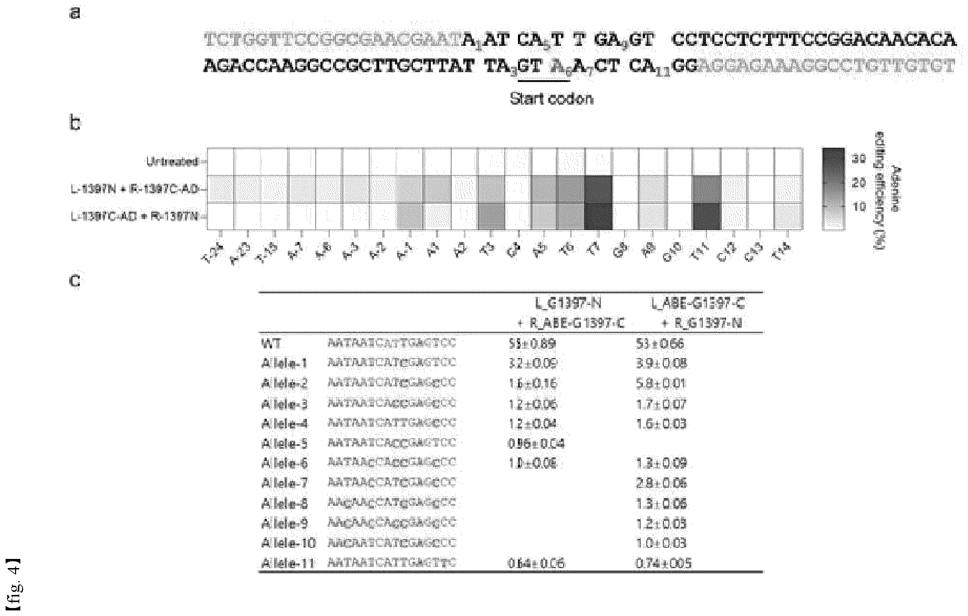

7 days after delivery of 30 µg of *in vitro-*synthesized mRNA to lettuce protoplasts, the efficiency of adenine base editing was confirmed. The results thereof are shown in FIG. 4. FIG. 4a shows binding of the left and right TALE arrays to the DNA sequence in blue. A5 and A6 are the target adenines of the start codon. According to FIG. 4b, the base editing rates of A5 are 10.67% and 7.68%, and those of A6 are 14.66% and 12.27%. According to FIG. 4c, adenine targeting WT is marked in blue, and the alleles in which targets are changed are Allele-3, Allele-5, Allele-6, and Allele-9.

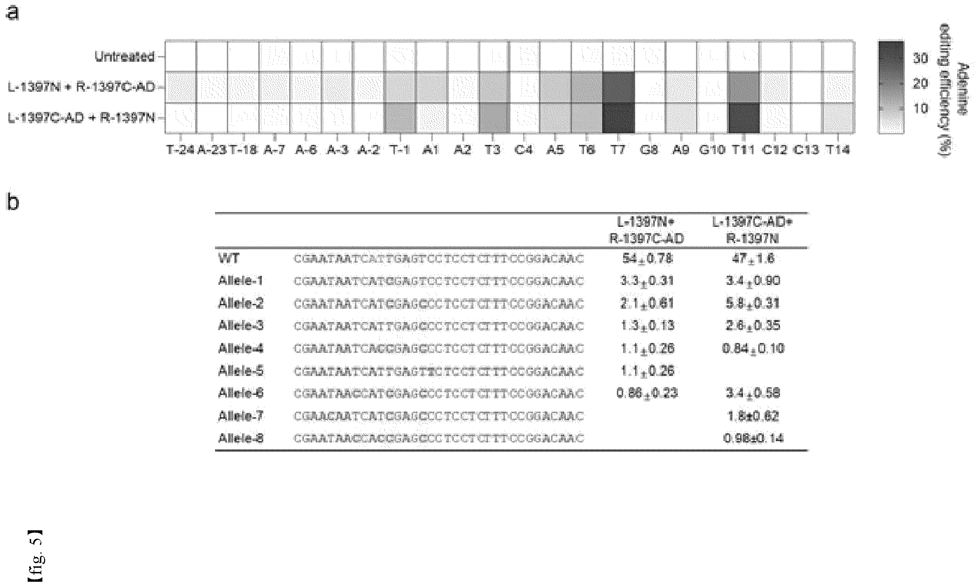

After callus was obtained through culture following mRNA delivery, the efficiency of adenine base editing was confirmed. According to FIG. 5a, the base editing rates of A5 are 8.4% and 7.5%, and those of A6 are 12.2% and 10.0%. According to FIG. 5b, adenine targeting WT is marked in blue, and the alleles in which targets are changed are Allele-4 and Allele-8.

### Example 3.

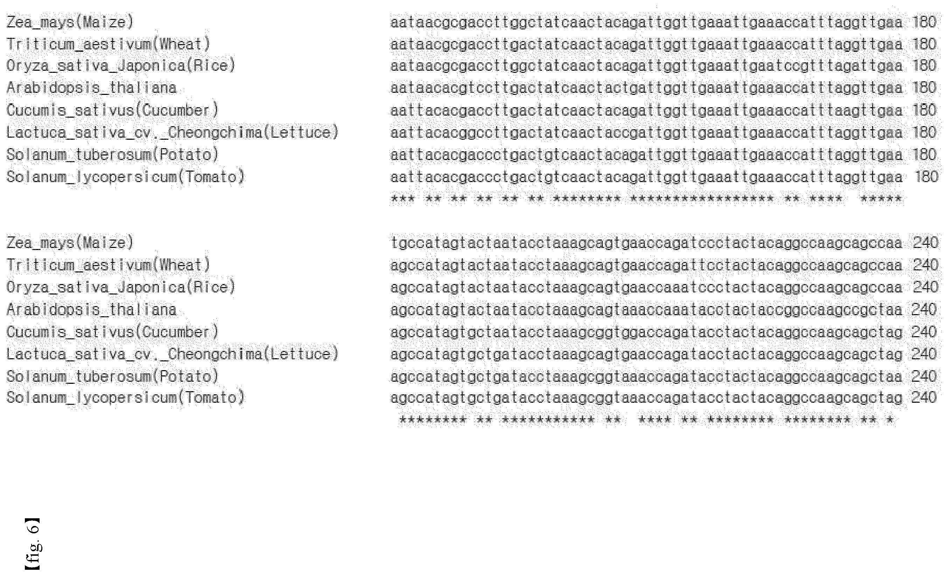

Plant chloroplast DNA has high homology. FIG. 6 shows the psbA DNA sequences of various plants, with the red a indicating the target position. Herbicide-resistant plants may be developed through base editing of chloroplast DNA of commercially valuable maize, tomato, potato, rice, wheat, and the like by methods using mRNA delivery, RNP, and agrobacterium. The sequences of Zea mays-NC_00166.2, Oryza sativa Japonica-NC_001320.1, Cucumis sativus-NC_007144, Triticum aestivum-NC_002762.1, Solanum tuberosum-NC_008096, Solanum lycopersicum-NC_007898, Arabidopsis Thaliana-AP000423, Lettuce sativa-AP007232.1 were used.

### [Industrial Applicability]

The only base editing of plant cell organelle DNA was the deamination of cytosine to thymine using DddAtox. This invention expands the scope of base editing of organelle DNA by base-editing adenine to guanine.

Although herbicide-resistant plants have been developed to date through base editing of DNA in the nucleus, base editing of psbA in chloroplasts has made it possible to develop new herbicide-resistant plants.

Development of new plant traits is possible through editing of not only psbA in chloroplasts but also other chloroplast genes.

Since base editing of the RuBisCO large unit involved in photosynthesis is possible, it is expected that plant production will increase by controlling photosynthetic efficiency.

Adenine in plant mitochondrial DNA can be edited using MTS (mitochondrial targeting signal) instead of CTS.

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

### [Sequence List Free Text]

An electronic file is attached.

## Claims

1. A composition for editing an adenine base of plant cell organelle DNA, comprising:
a DNA binding protein or a nucleic acid encoding the same;
a first split and a second split derived from a cytosine deaminase or a nucleic acid encoding the same; and
an adenine deaminase or a nucleic acid encoding the same,
wherein each of the first split and the second split binds to the DNA binding protein.

2. The composition according to claim 1, wherein each of the first split and the second split has no cytosine deaminase activity.

3. The composition according to claim 1, wherein the cytosine deaminase is derived from a deaminase (DddA) that acts on double-stranded DNA or an orthologue thereof.

4. The composition according to claim 1, wherein the first split comprises a sequence from N-terminus to at least one selected from the group consisting of G33, G44, A54, N68, G82, N98, and G108 in a sequence of SEQ ID NO: 1.

5. The composition according to claim 1, wherein the second split comprises a sequence from at least one selected from the group consisting of G34, P45, G55, N69, T83, A99, and A109 to C-terminus in a sequence of SED ID NO: 1.

6. The composition according to claim 1, wherein the DNA binding protein is a ZF protein (zinc finger protein) or a TALE array (transcription activator-like effector array).

7. The composition according to claim 1, wherein the DNA binding protein is linked via a peptide linker comprising 2 to 40 amino acid residues.

8. The composition according to claim 7, wherein the linker comprises:
2a.a linker: GS;
5a.a linker: TGEKQ;
10a.a linker: SGAQGSTLDF;
16a.a linker: SGSETPGTSESATPES;
24 a.a linker: SGTPHEVGVYTLSGTPHEVGVYTL; or
32a.a linker: GSGGSSGGSSGSETPGTSESATPESSGGSSGGS.

9. The composition according to claim 1, wherein the DNA binding protein is a zinc finger protein, and each of the first split and the second split binds to N-terminus or C-terminus of the zinc finger protein.

10. The composition according to claim 1, wherein the DNA binding protein is a TALE array, and a single TALE array binds to one terminus of a split or a first TALE array and a second TALE array bind to the first split and the second split.

11. The composition according to claim 1, wherein the adenine deaminase binds to N- or C-terminus of the DNA binding protein or the cytosine deaminase.

12. The composition according to claim 1, wherein the adenine deaminase is a deoxy-adenine deaminase derived from TadA.

13. The composition according to claim 1, comprising a chloroplast transit peptide or a nucleic acid encoding the same.

14. The composition according to claim 1, comprising a mitochondrial targeting signal (MTS) or a nucleic acid encoding the same.

15. The composition according to claim 1, which is delivered to a plant cell through:
gene gun bombardment;
PEG-mediated protoplast transfection;
protoplast transfection by electroporation; or
protoplast injection by microinjection.

16. The composition according to claim 1, wherein the nucleic acid is delivered to a plant cell through:
transformation using Agrobacterium comprising *Agrobacterium tumefaciens* or *Agrobacterium rhizogene;*
viral transfection;
gene gun bombardment;
PEG-mediated protoplast transfection;
protoplast transfection by electroporation; or
protoplast injection by microinjection.

17. A method of editing adenine to guanine in plant cell organelle DNA, comprising treating a plant cell with the composition according to any one of claims 1 to 16.

18. An atrazine herbicide-resistant plant in which an adenine base of a chloroplast psbA gene comprising a GTTGAAAGC sequence is edited to guanine by the method according to claim 17.

19. A spectinomycin-resistant plant in which an adenine base of 16s rRNA comprising a CGTCATCCTCA sequence is edited to guanine by the method according to claim 17.

20. A plant having an albino phenotype in which an adenine base of a psaA gene comprising ATG is edited to guanine by the method according to claim 17.
